# EUROPEAN PATENT SPECIFICATION

(11) **EP 1 796 651 B1**
(45) Date of publication and mention of the grant of the patent: **20.05.2015**
(21) Application number: 05789203.6
(22) Date of filing: 18.08.2005
(51) Int. Cl.: A61K 9/70

(54) **TRANSDERMAL DRUG DELIVERY DEVICE WITH TRANSLUCENT PROTECTIVE FILM**
TRANSDERMALE ARZNEIABGABEVORRICHTUNG MIT DURCHSCHEINENDER SCHUTZFOLIE
DISPOSITIF D'ADMINISTRATION DE MEDICAMENT TRANSDERMIQUE AVEC FILM PROTECTEUR TRANSLUCIDE

(30) Priority: 20.08.2004 US 603497 P
(43) Date of publication of application: 20.06.2007
(73) Proprietor: 3M Innovative Properties Company, St. Paul, MN 55133-3427 (US)
(72) Inventor: Wold, Chad R.,, Saint Paul, Minnesota 55133-3427 (US)
(74) Representative: Vossius & Partner Patentanwälte Rechtsanwälte mbB
(86) International application number: PCT/US2005/029397
(87) International publication number: WO 2006/023644

(56) References cited:
- EP-A- 1 269 999
- US-A1- 2003 194 382
- US-A1- 2005 177 086

## Description

The present invention relates to transdermal drug delivery devices. In particular, this invention relates to transdermal delivery devices with a protective film backing layer. The present invention also relates to methods of drug delivery using a transdermal drug delivery device with a protective film backing.

### Background of the Invention

Transdermal drug delivery is a well known method for administering pharmaceuticals. Transdermal drug delivery devices typically consist of a reservoir containing a drug. An example of such a reservoir is an adhesive matrix, or patch, that has a drug dispersed or dissolved throughout the matrix. The adhesive matrix is placed in contact with a skin surface when in use and the drug passes from the device into and through the skin. Additional excipients, such as skin permeation enhancers or drug solubilizers may also be incorporated into the reservoir. Such devices typically have a film backing material that protects the portion of the reservoir that is not in contact with the skin.

Typical protective film backings used in transdermal devices, such as laminates including aluminum foil, are opaque. In some cases, translucent patch backings are used so that they may be worn on an exposed area on the body and be visually unobtrusive. Typical flexible and translucent materials used in transdermal devices, are polyethylene films.

### Summary of the Invention

However, translucent backings can have limitations in their ability to protect against ultraviolet exposure. Certain drugs are sensitive to ultraviolet light exposure and may undergo degradation while being worn by a patient, particularly if worn on an exposed area. It has now been found that transdermal drug delivery devices may be prepared by adding relatively small amounts of inorganic ultraviolet-absorbing compounds to the protective film backing of the device to protect a pharmaceutical formulation according to claim 1.

EP 1 269 999, US 2003/194382, JP 61 221119, Japanese Utility Model No. 5-30118 reveal patches, plasters with ultraviolet-absorbing pigments, but without a translucent film/backing sheet.

Devices of the present invention are advantageous, since the inorganic ultraviolet-absorbing compound resist diffusing from the translucent film, thus prevent substantial contact with drug that is present in the reservoir and reduces any likelihood of chemical degradation induced by an ultraviolet absorbing additive.

Additionally, the formulation is protected from ultraviolet light without introduction of additives that may adversely interact with the pharmaceutical formulation.

In one aspect, the present invention provides a transdermal drug delivery device including a reservoir together with a releasably stored dosage of a pharmaceutically active agent and a translucent film in relation to at least a portion of the reservoir, wherein the translucent film comprises at least one inorganic ultraviolet-absorbing compound.

In another aspect, the present invention can provide a method of drug delivery to a mammal that will protect and prevent the formulation from degradation by a process of providing a reservoir including a pharmaceutically active agent and a translucent backing film adjacent to at least a portion of the reservoir, wherein the backing film comprises at least one inorganic ultraviolet-absorbing compound, and placing the surface of the reservoir opposed to the backing film in a delivering relationship to the skin surface of the mammal which will allow the reservoir to remain in a delivering relationship to the skin for a period of time sufficient to provide a therapeutic effect.

One benefit of this invention is the presence of inorganic ultraviolet absorbing substances in the backing. Small molecule compounds, such as conventional organic ultraviolet absorbing substances, can diffuse relatively easily within polymeric matrices. Thus a small molecule blended into a polymeric film, such as for example, polyethylene, will generally diffuse within the film. A small molecule will also generally diffuse into any layers adjoining the film, such as a pressure sensitive adhesive layer. This may be disadvantageous, particularly where the pressure sensitive adhesive layer also serves as a drug reservoir (i.e., a "drug-in-adhesive" system). Any small molecules present within a pressure sensitive adhesive drug reservoir may be prone to contacting the skin of a patient or subsequently diffusing through the skin and into the systemic circulation.

Also, interactions between conventional organic ultraviolet absorbing substances and a drug may lead to undesirable effects such as chemical degradation of the drug or binding of the drug to the pressure sensitive adhesive matrix. Additionally, loss of effective dose may be an additional drawback of organic ultraviolet absorbing substances.

### Brief Description of the Drawings

Preferred embodiments of the invention will now be described in greater detail below with reference to the attached drawings, wherein:
FIG. 1 is a schematic cross-section of an embodiment of the present invention where the device has a reservoir comprising a pressure-sensitive adhesive.
FIG. 2 is a schematic cross-section of an embodiment of the present invention where the device has a reservoir enclosed by a film backing and a membrane.
FIG. 3 is a schematic cross-section of an embodiment of the present invention where the device has a peripheral ring of skin-contact adhesive surrounding the reservoir.
FIG. 4 is ultraviolet-visible absorbance spectra for the films of Examples 1 to 4 and Comparative Example 1.

### Detailed Description

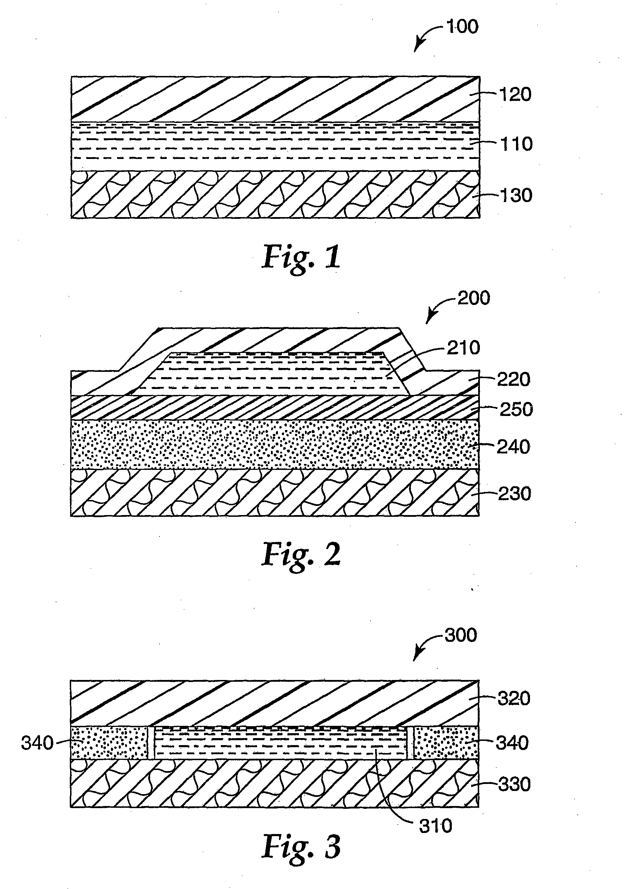

In the embodiment shown in FIG. 1, the transdermal drug delivery device 100 has a reservoir 110 comprising a pharmaceutically active agent an opposing pressure-sensitive adhesive. The translucent film backing 120 directly contacts the reservoir 110. The device 100 further has a release liner 130 proximate the opposing surface of the reservoir 110 to protect the skin-contacting surface prior to application on a patient. This is referred to as a "drug-in-adhesive" patch.

Suitable transdermal drug delivery devices 100 include gelled or liquid reservoirs, such as in U. S. Patent No. 4,834,979 (Gale), so-called "reservoir" patches; devices containing matrix reservoirs attached to the skin by an adjacent adhesive layer, such as in U. S. Patent No. 6,004,578 (Lee, et al.), so-called "matrix" patches; and devices containing pressure-sensitive adhesive reservoirs, such as in U. S. Patent Nos. 6,365,178 (Venkateshwaran et al.), 6,024,976 (Miranda et al.), and 6,149,935 (Chiang et al.), so-called "drug-in-adhesive" patches, the disclosures of which are incorporated herein by reference.

The length of time that the reservoir 110 remains in a delivering relationship is typically an extended time, for example, from about 12 hours to about 14 days. In certain embodiments, the length of time that the reservoir remains in a delivering relationship is about 1 day (i.e., daily dosing), about 3 to 4 days (bi-weekly dosing), or about 7 days (weekly dosing).

In one embodiment, the reservoir 110 may contain other additives or excipients in addition to the pharmaceutically active agent. Such additives include pharmaceutically acceptable materials that may be used as skin penetration enhancers (i.e., substances that increase the permeation rate of a drug across or into the skin) or solubilizers (i.e., substances that effectively solubilize a drug) in transdermal drug delivery systems. Suitable materials used as skin permeation enhancers include C₈-C₂₀ fatty acids such as isostearic acid, octanoic acid, and oleic acid; C₈-C₂₀ fatty alcohols such as oleyl alcohol and lauryl alcohol; lower alkyl esters of C₈-C₂₀ fatty acids such as ethyl oleate, isopropyl myristate, butyl stearate, and methyl laurate; di(lower) alkyl esters of C₆-C₈ diacids such as diisopropyl adipate; monoglycerides of C₈-C₂₀ fatty acids such as glyceryl monolaurate; tetraglycol (tetrahydrofurfuryl alcohol polyethylene glycol ether); tetraethylene glycol (ethanol,2,2'-(oxybis(ethylenoxy))diglycol); C₆-C₂₀ alkyl pyrrolidone carboxylates; polyethylene glycol; propylene glycol; 2-(2-ethoxyethoxy)ethanol; diethylene glycol monomethyl ether; N,N-dimethyldodecylamine-N-oxide and combinations of the foregoing. Alkylaryl ethers of polyethylene oxide, polyethylene oxide monomethyl ethers, polyethylene oxide dimethyl ethers, glycerol, and N-methyl pyrrolidone are also suitable. The terpenes are another useful class of pharmaceutical excipients, including pinene, d-limonene, carene, terpineol, terpinen-4-ol, carveol, carvone, pulegone, piperitone, menthone, menthol, neomenthol, thymol, camphor, borneol, citral, ionone, and cineole, alone or in any combination. Examples of other additives include tackifiers, plasticizers, and anti-oxidants.

Exemplary pharmaceutically active agents (also referred to as "drugs") that can be included in the reservoir 110 are capable of local or systemic effect when administered to the skin. Some examples include, clonidine, estradiol, nicotine, nitroglycerine, scopolamine, and fentanyl, which are commercially available in the form of transdermal devices. Other examples include antiinflammatory drugs, both steroidal (e.g., hydrocortisone, prednisolone, triamcinolone) and nonsteroidal (e.g., naproxen, piroxicam); bacteriostatic agents (e.g., chlorhexidine, hexylresorcinol); antibacterials (e.g., penicillins such as penicillin V, cephalosporins such as cephalexin, erythromycin, tetracycline, gentamycin, sulfathiazole, nitrofurantoin, and quinolones such as norfloxacin, flumequine, and ibafloxacin); antiprotazoals (e.g., metronidazole); antifungals (e.g., nystatin); coronary vasodilators; calcium channel blockers (e.g., nifedipine, diltiazem); bronchodilators (e.g., theophylline, pirbuterol, salmeterol, isoproterenol); enzyme inhibitors such as collagenase inhibitors, protease inhibitors, elastase inhibitors, lipoxygenase inhibitors (e.g., A64077), and angiotensin converting enzyme inhibitors (e.g., captopril, lisinopril); other antihypertensives (e.g., propranolol); leukotriene antagonists (e.g., ICI204,219); anti-ulceratives such as H2 antagonists; steroidal hormones (e.g., progesterone, testosterone, estradiol); antivirals and/or immunomodulators (e.g., 1-isobutyl-1H-imidazo[4,5-c]quinolin-4-amine, 1-(2-hydroxy-2-methylpropyl)-1H-imidazo[4,5-c]quinolin-4-amine, and acyclovir); local anesthetics (e.g., benzocaine, propofol); cardiotonics (e.g., digitalis, digoxin); antitussives (e.g., codeine, dextromethorphan); antihistamines (e.g., diphenhydramine, chlorpheniramine, terfenadine); narcotic analgesics (e.g., morphine, buprenorphine); peptide hormones (e.g., human or animal growth hormones, LHRH); cardioactive products such as atriopeptides; proteinaceous products (e.g., insulin); enzymes (e.g., anti-plaque enzymes, lysozyme, dextranase); antinauseants; anticonvulsants (e.g., carbamazine); immunosuppressives (e.g., cyclosporine); psychotherapeutics (e.g., diazepam); sedatives (e.g., phenobarbital); anticoagulants (e.g., heparin); analgesics (e.g., acetaminophen); antimigraine agents (e.g., ergotamine, melatonin, sumatripan); antiarrhythmic agents (e.g., flecainide); antiemetics (e.g., metaclopromide, ondansetron); anticancer agents (e.g., methotrexate); neurologic agents such as anxiolytic drugs; hemostatics; anti-obesity agents; and the like, as well as pharmaceutically acceptable salts and esters thereof. The amount of drug that constitutes a therapeutically effective amount can be readily determined by those skilled in the art with due consideration of the particular drug, the particular carrier, and the desired therapeutic effect. This invention is particularly useful for drugs that are sensitive to ultraviolet light. This may include, for example, compounds containing one or more ultraviolet absorbing functional groups, such as a carbon-carbon double bond, carbonyl, or aromatic group. Examples of ultraviolet light sensitive drugs include nicotine, antibiotics, calcium channel blockers (i.e., ifedipine, felodipine, and lacidipine), vitamins (i.e., B12), and progestins (i.e., norgestimate, and levonorgestrel).

In the present invention, a translucent film backing 220 including an inorganic ultraviolet-absorbing compound may be made by any conventional means of forming a film. The translucent film together with at least one inorganic ultraviolet-absorbing compound. In one embodiment, the translucent film is substantially free of organic ultraviolet-absorbing compounds beyond those present in the base constituents of the film. Suitable inorganic ultraviolet-absorbing compounds include titanium dioxide, zinc oxide, and iron oxide. In one embodiment, titanium dioxide is an inorganic ultraviolet-absorbing compound. These compounds are typically provided in particulate form and may be in the form of a powder, flakes, needles, or granules. Additionally, the compounds may also be present as single particles, as agglomerates of several particles, or as a mixture of single particles and agglomerates.

The inorganic ultraviolet absorbing compound may be handled and processed in a dry powder form, as a dispersion in a polymeric matrix and as a dispersion in a liquid medium by any other suitable conventional means for inclusion in the translucent film. The particles or agglomerates are sufficiently small so as to avoid excessive absorption of visible light. The average particle size is preferably smaller than about 100 nanometers, more preferably smaller than about 50 nanometers, and most preferably smaller than about 25 nanometers. Although there is no lower bound on the particle or agglomerate size with regard to the scattering or absorption of visible light, the average particle size is typically larger than about 1 nanometer. Such small inorganic particles may be manufactured by a variety of conventional methods, such as those disclosed in U.S. Patent Nos. 6,653,356 (Sherman), 6,440,383 (Duyvesteyn et al.), and 5,840,111 (Wiederhoft et al.). The inorganic ultraviolet-absorbing compound may be present in a form such that it does not migrate or diffuse within the translucent film once the film has been prepared. Particles and agglomerates, as described above, are too large to undergo diffusion or migration as compared to an organic absorber or a single molecule within a polymeric film that is maintained under ordinary temperature conditions.

In one embodiment, the inorganic ultraviolet-absorbing compound is typically inert with respect to the components of the reservoir 110, such as the pharmaceutically active agent and any other excipients present in the reservoir 110. However, compounds that are not entirely inert may be effectively employed as long as they have limited or no ability to migrate or diffuse from the film. In such a situation, the compound may be encapsulated within the film and will not adversely affect sensitive compounds within the reservoir 110.

The inorganic ultraviolet-absorbing compound may be homogenously dispersed within the translucent film. In one embodiment, the inorganic ultraviolet-absorbing compound may be present at the surfaces of the film, and substantially all of the inorganic ultraviolet-absorbing compound is encapsulated within the film and is not exposed at the outer surfaces of the film. Particulate inorganic compounds may optionally comprise one or more thin surface coatings to aid with powder flow, dispersability, and/or reduction in photoactivity. Examples of such surface coatings include inorganic coatings, such as oxides, hydroxides, or hydrous oxides of aluminum, silicon, zirconium, tin, magnesium, zinc, cerium, and phosphorous, and organic coatings, such as stearic acid and derivatives thereof.

Chemical stability of the transdermal delivery devices may be measured by preparing devices of the invention, storing them under conditions of 25°C and 60% relative humidity, and testing the devices for concentration of one or more pharmaceutically active agents (or drugs) at predetermined storage times. In one embodiment, the amount of a drug is more than about 95% by weight, preferably more than about 97% by weight of the initial amount of the drug in the device 100 when stored at 25°C and 60% relative humidity for a period of 6 months. In another embodiment, the amount of a drug is more than about 95%, preferably more than about 97%, by weight of the initial amount of the drug in the device 100 when stored at 25°C and 60% relative humidity for a period of time of 1 year.

Accelerated chemical stability may be measured by preparing devices of the invention, storing them under conditions of 40°C and 75% relative humidity, and testing the devices for concentration of one or more drugs at predetermined storage times. In one embodiment, the amount of a drug is more than about 95%, preferably more than about 97%, by weight of the initial amount of the drug in the device when stored at 40°C and 75% relative humidity for a period of time of 3 months, and more than about 90%, preferably more than about 93%, by weight of the initial amount of the drug in the device when stored for a period of time of 6 months.

The concentration of inorganic ultraviolet-absorbing compound required will depend on several factors including: the refractive index of the compound, particle size, particle shape, refractive index of the base constituents of the translucent film, film thickness, desired ultraviolet absorbency, and desired visible light translucency. High concentrations of such particles in a film can lead to unacceptable decreases in overall translucency of the film. Typical concentrations of inorganic ultraviolet-absorbing compound will be less than about 10 percent, often less than about 5 percent, and sometimes less than about 2.5 percent, by weight of the total weight of the translucent film. Typical concentrations of inorganic ultraviolet-absorbing compound will be more than about 0.05 percent, often more than about 0.1 percent, and sometimes more than about 0.25 percent, by weight of the total weight of the translucent film.

In one embodiment, the present invention comprises a translucent film that is substantially free of organic ultraviolet-absorbing compounds beyond those present in the base constituents of the film. It should be understood that the base, polymeric constituents of the translucent film are organic compounds that may have some ability to absorb ultraviolet light. By substantially free, it is understood that this means essentially no additional organic ultraviolet-absorbing compounds are added to the base constituents of the translucent film, such as benzotriazoles or benzophenones, are added to enhance the ultraviolet absorbency of the translucent film. In this embodiment, any organic ultraviolet-absorbing compounds within the translucent film that are added to the base constituents of the film are present only in insignificant amounts, such as might be present as a trace impurity or as a minor process additive used in a raw material, such as in the polymeric pellets used to form the film. By insignificant amounts, it is understood that the increase in ultraviolet absorbance beyond the inherent absorbance of the base constituents of the translucent film will be primarily due to the inorganic ultraviolet-absorbing compounds of the present invention. Usually more than 95 percent and in some cases more than 99 percent of the increased ultraviolet absorbance beyond the inherent absorbance of the base constituents of the translucent film will be due to the inorganic ultraviolet-absorbing compounds.

In the embodiment shown in FIG. 2, the device 200 has a reservoir 210 comprising a therapeutically active agent. The reservoir 210 is adjoined and protected by a translucent film backing 220. The reservoir 210 also adjoins a membrane 250, which together with the film backing 220 encloses the reservoir 210. Thus, the reservoir 210 may be a liquid or gel formulation, since it is enclosed by the backing 220 and membrane 250. The membrane is adjoined by a skin contact adhesive layer 240, which is used to affix the device 200 to a skin surface. The device 200 further has a release liner 230 to protect the skin-contacting surface of the skin contact adhesive layer 240 prior to application on a patient. As noted above, this is a so-called "reservoir" patch.

A translucent film backing 220 comprising an inorganic ultraviolet-absorbing compound may be made by any conventional means of forming a film. One such example would be to add pellets of a polymeric material and particles of an inorganic ultraviolet-absorbing compound to a mixing device, such as a single- or twin-screw extruder. The extruder both melts the polymeric material and mixes the polymeric material with the inorganic ultraviolet-absorbing compound until a homogeneous, molten mixture is prepared. The homogeneous, molten mixture is then forced through an extrusion die to form a flat sheet of polymeric material that may be cooled on a chilled surface or air-cooled. Prior to cooling, the film may be stretched in either the machine and/or transverse direction to reduce the film thickness (or caliper) and/or induce orientation within the film.

In another embodiment, particles of an inorganic ultraviolet-absorbing compound may be mixed or blended with pellets of a polymeric material to form a "pre-" or "master-" batch of resin mixed with the inorganic ultraviolet-absorbing compound prior to compounding into a flat sheet of polymeric material. This master-batch may have a higher concentration of inorganic ultraviolet-absorbing compound than is desired in the final product, and may, for example, be blended with additional pellets of polymeric material in a mixing device to form a homogeneous, molten mixture having the desired concentration of inorganic ultraviolet-absorbing compound. As described above, the homogeneous, molten mixture may be formed into a film. Alternatively, the master-batch may be prepared with at the desired concentration of inorganic ultraviolet-absorbing compound for the final product, in which case the compounded pellets of the master-batch need only be formed into a flat sheet by any conventional processing method, such as with the use of a single-screw extruder and an extrusion die.

The term translucent means that the translucent film permits the passage of light. That is, in one embodiment, such a film may be clear or transparent. Alternatively, it may transmit sufficient light such that an object on the other side of the film may be observed, but not clearly seen. Although there is no precisely defined range of wavelengths encompassing visible light due to the variation in human perception, for definitional purposes the visible spectrum will be considered to be between 400 and 700 nanometers.

Typical examples of flexible films employed as conventional tape backings which may be useful for the present invention include those made from polymer films such as polypropylene; polyethylene, particularly low density polyethylene, linear low density polyethylene, metallocene polyethylenes, medium density polyethylene, and high density polyethylene; polyvinyl chloride; polyester (e.g., polyethylene terephthalate); polyvinylidene chloride; polyethylene copolymers, such as ethylene-vinyl acetate copolymer; styrenic block copolymers, such as styrene-ethylene/butylene copolymers; polyurethane; cellulose acetate; and ethyl cellulose. Coextruded multilayer polymeric films are also suitable and may, for example, comprise one or more of the above mentioned polymers. Additional examples of multilayer polymeric films are described in U. S. Patent No. 5, 783, 269 (Heilmann et al.), and U. S. Patent Application Publication No. 2004/0219198 (Johnson et al.).

Polyethylenes, polyethylene blends, and polypropylenes are preferred polymer films. Polyethylenes, polyethylene copolymers, and polyethylene blends are most preferred polymer films. Additives may also be added to the translucent film, such as tackifiers, plasticizers, colorants, and anti-oxidants.

In one embodiment, the translucent film thickness is more than 10 µm, typically more than 20 µm, and usually more than 40 µm. In another embodiment, the translucent film thickness is less than 150 µm, typically less than 125 µm, and usually less than 100 µm.

Ultraviolet and/or light absorbance of the translucent film may be measured using a standard ultraviolet-visible spectrophotometer by mounting the film sample in the sample holder at a 90 degree angle to the incident beam and subsequently measuring the film absorbance as a function of incident wavelength. The ultraviolet spectrum of interest encompasses the range between 200 and 400 nanometers. It is generally desired to have a low absorbance of the translucent film in the visible spectrum to allow significant light transmission. It is also generally desired that the absorbance of a translucent film comprising the inorganic ultraviolet-absorbing compound is not substantially higher than the absorbance of a like translucent film without an inorganic ultraviolet-absorbing compound.

In one embodiment, the absorbance of the translucent film at a wavelength of 500 nanometers is less than about 1.0 absorbance unit, preferably less than about 0.5 absorbance units more than the absorbance of a like film not comprising an inorganic ultraviolet-absorbing compound. In one embodiment, the absorbance of the translucent film is less than about 2.5 absorbance units, and in come cases less than about 2.0 absorbance units at a wavelength of 500 nanometers.

In one embodiment, the absorbance of the translucent film at a wavelength of 450 nanometers is less than about 2 absorbance units. In one embodiment, the absorbance of the translucent film at a wavelength of 450 nanometers is less than about 1.0 absorbance units, and in some cases less than about 0.5 absorbance units more than the absorbance of a like translucent film without an inorganic ultraviolet-absorbing compound.

The translucent film with the inorganic ultraviolet absorbing compound will absorb some portion of ultraviolet light and preferably absorb more ultraviolet light than a like film that does not comprise an inorganic ultraviolet-absorbing compound. In one embodiment, the absorbance of the translucent film is more than about 3.0 absorbance units at a wavelength of 350 nanometers. In one embodiment, the absorbance of the translucent film is more than about 3.0 at a wavelength of 300 nanometers.

In one embodiment, the absorbance of the translucent film at a wavelength of 300 nanometers is at least about 0.5 absorbance units, typically at least about 1.0 absorbance units, more than the absorbance of a like film not including an inorganic ultraviolet-absorbing compound. In one embodiment, the absorbance of the translucent film at a wavelength of 350 nanometers is at least about 0.5 absorbance units, typically at least about 1.0 absorbance units, more than the absorbance of a like film not including an inorganic ultraviolet-absorbing compound.

Typical organic materials used as the base constituents of polymeric films are opaque to radiation at wavelengths below 200 nanometers. In one embodiment, the absorbance of the translucent film is more than about 3.0 absorbance units at all wavelengths between 200 and 300 nanometers. In one embodiment, the absorbance of the translucent film is more than about 3.0 absorbance units at all wavelengths between 200 and 350 nanometers.

Transdermal drug delivery devices of the invention can be made in the form of an article such as a tape, a patch, a sheet, a dressing or any other form known to those skilled in the art. Generally, the device will be in the form of a patch of a size suitable to deliver a selected amount of drug through the skin.

Generally, the device will have a surface area greater than about 1 cm², and more typically greater than about 5 cm². Generally, the device will have a surface area of less than about 100 cm², often less than about 40 cm², and sometimes less than about 20 cm². Devices of the present invention may be packaged individually in a foil-lined pouch for storage. Devices of the present invention may alternatively be provided in a rolled or stacked form suitable for use with a dispensing apparatus.

In the embodiment shown in FIG. 3, the device 100 is similar to that shown in FIG. 1, except that the reservoir 310 may or may not be a skin-adhesive layer. A peripheral ring of skin-contact adhesive 340 is provided to ensure that the device will adhere to a skin surface. A release liner 330 protects the skin-contacting surfaces of the skin contact adhesive layer 340 and the reservoir 310 prior to application on a patient. This is a so-called "matrix" patch.

In one aspect, devices 300 of the present invention comprise a release liner 330 that covers and protects the skin-contacting surface prior to use by a patient. Suitable release liners include conventional release liners comprising a known sheet material such as a polyester web, a polyethylene web, a polypropylene web, or a polyethylene-coated paper coated with a suitable fluoropolymer or silicone based coating.

### Examples

### Example 1

A translucent film suitable for use in a transdermal drug delivery device was prepared according to the following general procedure. Titanium dioxide coated with aluminum oxide and stearic acid (Hombitec® RM 130F available from Sachtleben Chemie GmbH, Duisburg, Germany) with a nominal crystallite size of 15 nanometers was used as the inorganic ultraviolet-absorbing compound. This material was purchased as a 20% by weight titanium dioxide concentrate dispersed within polyethylene.

The 20% titanium dioxide concentrate was mixed with low-density polyethylene (LDPE, NA 964-085 NT available from Equistar, Morris, Illinois) at a 1:39 ratio in a single-screw extruder and extruded into a 3 mil (76 µm) thick film having a matte finish. The resulting film had a concentration of 0.5 percent titanium dioxide by weight.

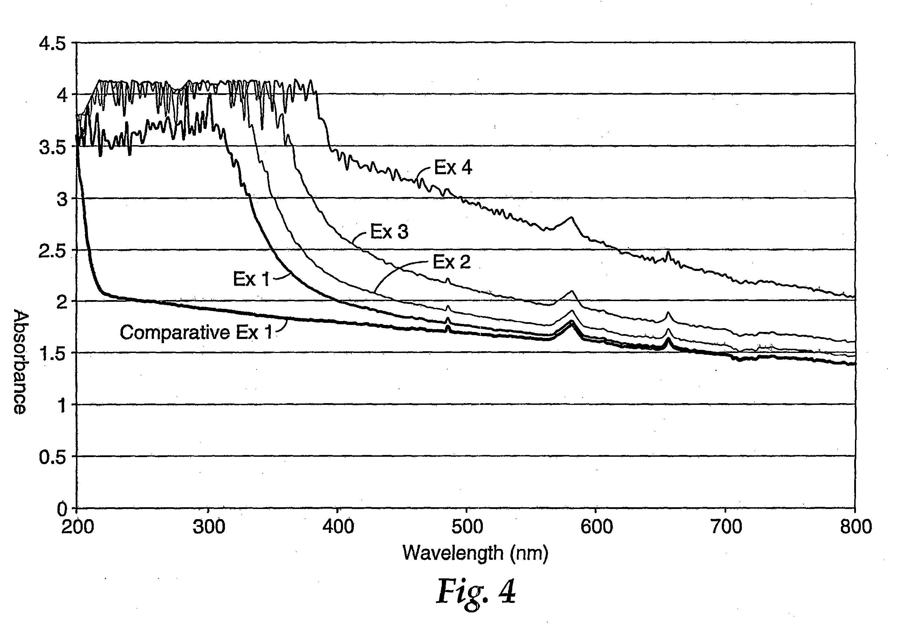

The ultraviolet-visible absorption spectrum of the film was measured using a Hewlett-Packard 8452A diode array ultraviolet-visible absorption spectrophotometer. The film sample was mounted in the sample chamber at a normal incidence angle to the beam and measured over a scan range of 200 to 800 nm at room temperature using air as a blank reference. The measured ultraviolet-visible absorption spectrum is shown in Figure 4.

### Example 2

A translucent film was prepared according to the general procedure of Example 1 with the exception that the low-density polyethylene/ titanium dioxide blend was further mixed with additional low-density polyethylene at a 1:19 ratio to produce a film with a concentration of 1.0 percent by weight titanium dioxide. The measured ultraviolet-visible absorption spectrum is shown in Figure 4.

### Example 3

A translucent film was prepared according to the general procedure of Example 1 with the exception that the low-density polyethylene/ titanium dioxide blend was further mixed with additional low-density polyethylene at a 1:9 ratio to produce a film with a concentration of 2.0 percent by weight titanium dioxide. The measured ultraviolet-visible absorption spectrum is shown in Figure 4.

### Example 4

A translucent film was prepared according to the general procedure of Example 1 with the exception that the low-density polyethylene/ titanium dioxide blend was further mixed with additional low-density polyethylene at a 1:3 ratio to produce a film with a concentration of 5.0 percent by weight titanium dioxide. The measured ultraviolet-visible absorption spectrum is shown in Figure 4.

### Comparative Example 1

A translucent film was prepared according to the general procedure of Example 1 with the exception that no titanium dioxide was added. The measured ultraviolet-visible absorption spectrum is shown in Figure 4.

### Example 5

A translucent polyethylene film was prepared according to the general procedure of Example 1. A transdermal adhesive formulation was prepared by mixing isopropanol, levonorgestrel, estradiol, an acrylate copolymer adhesive, and Kollidon® VA 64. The resulting adhesive formulation was knife coated onto a fluoropolymer-coated polyester release liner (ScotchPak™ 1022) and dried to provide an adhesive layer with a coating weight of approximately 10 mg/cm². Drug concentration was measured by high performance liquid chromatography (HPLC) and is reported as the ratio of drug to total adhesive formulation on a weight-weight basis. The average concentration of estradiol in samples not exposed to UV radiation was 20.45 mg per gram of adhesive. The average concentration of levonorgestrel in samples not exposed to UV radiation was 6.59 mg per gram of adhesive formulation. The side of the adhesive layer opposed to the release liner was laminated to the translucent polyethylene film (or backing) to prepare a 3-layer laminate (backing-adhesive- liner) suitable for use as a transdermal drug delivery device.

Samples of the 3-layer laminate with dimensions of 2 inch (5.08 cm) x 8 inch (20.32 cm) were exposed to UV radiation and subsequently tested for drug concentration. UV-visible exposure was performed with an Atlas SunTest CPS+-using a 300-800 nm irradiance of 450 W/m² with the Option 1 filters described in International Conference on Harmonisation of Technical Requirements for Registration of Pharmaceuticals for Human Use (ICH) quality document Q1B for a time period of 10 hours to obtain an illumination of not less than 1.2 Megalux hours and 200 watt hours of 300-400 nm UV exposure. Drug concentrations after exposure to UV radiation are reported in Table 1. In addition, the percentage reduction in drug concentration after UV exposure is also reported.

| Table 1 - Drug concentration after UV exposure | | | | | |
|---|---|---|---|---|---|
| | | Estradiol | | Levonorgestrel | |
| Example No. | % TiO₂ Conc. In Film | Concentration [mg/g] | % Reduction | Concentration [mg/g] | % Reduction |
| 5 | 0.5 | 19.42 | 5.0 | 5.38 | 18.4 |
| 6 | 1.0 | 19.77 | 3.3 | 5.92 | 10.1 |
| 7 | 2.0 | 20.19 | 1.3 | 6.38 | 3.2 |
| C2 | 0 | 18.75 | 8.3 | 4.34 | 34.1 |

### Example 6

A 3-layer laminate (backing-adhesive- liner) suitable for use as a transdermal drug delivery device was prepared according to Example 5, with the exception that the translucent polyethylene film was prepared according to the general procedure of Example 2.

### Example 7

A 3-layer laminate (backing-adhesive- liner) suitable for use as a transdermal drug delivery device was prepared according to Example 5, with the exception that the translucent polyethylene film was prepared according to the general procedure of Example 3.

### Comparative Example 2

A 3-layer laminate (backing-adhesive- liner) suitable for use as a transdermal drug delivery device was prepared according to Example 5, with the exception that the translucent polyethylene film was prepared according to the general procedure of Comparative Example 1.

## Claims

1. A transdermal drug delivery device comprising:
a) a reservoir comprising a releasably stored dosage of a pharmaceutically active agent; and
b) a translucent film in relation to at least a portion of the reservoir, wherein the translucent film comprises at least one inorganic ultraviolet-absorbing compound in a particulate form having an average particle size of 1 to 50 nanometers.

2. A transdermal drug delivery device according to claim 1, wherein the reservoir is a pressure sensitive adhesive.

3. A transdermal drug delivery device according to any one of claims 1 or 2, wherein the translucent film is a backing film adjoining the reservoir.

4. A transdermal drug delivery device according to any one of the preceding claims, wherein the translucent film is substantially free of organic ultraviolet-absorbing compounds beyond those present in the base constituents of the film.

5. A transdermal drug delivery device according to any one of the preceding claims wherein the inorganic ultraviolet-absorbing compound is selected from the group consisting of titanium dioxide, zinc oxide, and iron oxide.

6. A transdermal drug delivery device according to claim 1, wherein the concentration of inorganic ultraviolet-absorbing compound is less than 1 percent by weight of the total weight of the translucent film.

7. A transdermal drug delivery device according to any one of the preceding claims, wherein the translucent film comprises polyethylene, polyethylene copolymers, or polyethylene blends.

8. A transdermal drug delivery device according to any one of the preceding claims, wherein the translucent film absorbance at a wavelength of 500 nanometers is less than 2 absorbance units.

9. A transdermal drug delivery device according to any one of the preceding claims, wherein the translucent film absorbance at a wavelength of 300 nanometers is more than 3 absorbance units

## Patentansprüche

1. Vorrichtung zur transdermalen Arzneimittelfreisetzung, die Folgendes umfasst:
a) einen Sammelbehälter, der eine freisetzbar gespeicherte Dosis eines pharmazeutisch wirksamen Mittels umfasst; und
b) eine durchsichtige Folie in Verbindung mit mindestens einem Abschnitt des Sammelbehälters, wobei die durchsichtige Folie mindestens eine anorganische, ultraviolettes Licht absorbierende Verbindung in Teilchenform mit einer durchschnittlichen Teilchengröße von 1 bis 50 Nanometern umfasst.

2. Vorrichtung zur transdermalen Arzneimittelfreisetzung nach Anspruch 1, wobei der Sammelbehälter ein druckempfindlicher Klebstoff ist.

3. Vorrichtung zur transdermalen Arzneimittelfreisetzung nach einem der Ansprüche 1 oder 2, wobei die durchsichtige Folie eine Verstärkungsfolie ist, die an den Sammelbehälter angrenzt.

4. Vorrichtung zur transdermalen Arzneimittelfreisetzung nach einem der vorstehenden Ansprüche, wobei die durchsichtige Folie im Wesentlichen frei von organischen, ultraviolettes Licht absorbierenden Verbindungen außer den in den Basisbestandteilen der Folie vorliegenden ist.

5. Vorrichtung zur transdermalen Arzneimittelfreisetzung nach einem der vorstehenden Ansprüche, wobei die anorganische, ultraviolettes Licht absorbierende Verbindung ausgewählt ist aus der Gruppe bestehend aus Titandioxid, Zinkoxid und Eisenoxid.

6. Vorrichtung zur transdermalen Arzneimittelfreisetzung nach Anspruch 1, wobei die Konzentration an anorganischer, ultraviolettes Licht absorbierender Verbindung weniger als 1 Gewichtsprozent des Gesamtgewichts der durchsichtigen Folie beträgt.

7. Vorrichtung zur transdermalen Arzneimittelfreisetzung nach einem der vorstehenden Ansprüche, wobei die durchsichtige Folie Polyethylen, Polyethylencopolymere oder Polyethylenmischungen umfasst.

8. Vorrichtung zur transdermalen Arzneimittelfreisetzung nach einem der vorstehenden Ansprüche, wobei das Absorptionsmaß der durchsichtigen Folie bei einer Wellenlänge von 500 Nanometern weniger als 2 Absorptionseinheiten beträgt.

9. Vorrichtung zur transdermalen Arzneimittelfreisetzung nach einem der vorstehenden Ansprüche, wobei das Absorptionsmaß der durchsichtigen Folie bei einer Wellenlänge von 300 Nanometern mehr als 3 Absorptionseinheiten beträgt.

## Revendications

1. Dispositif d'administration transcutanée de médicament, comprenant :
a) un réservoir comprenant un dosage stocké de manière libérable d'un agent actif sur le plan pharmaceutique ; et
b) un film translucide en relation avec au moins une partie du réservoir, dans lequel le film translucide comprend au moins un composé inorganique absorbeur d'ultraviolets sous une forme particulaire possédant une taille moyenne de particules de 1 à 50 nanomètres.

2. Dispositif d'administration transcutanée de médicament selon la revendication 1, dans lequel le réservoir est un adhésif sensible à la pression.

3. Dispositif d'administration transcutanée de médicament selon l'une quelconque des revendications 1 ou 2, dans lequel le film translucide est un film de support attenant au réservoir.

4. Dispositif d'administration transcutanée de médicament selon l'une quelconque des revendications précédentes, dans lequel le film translucide est essentiellement dépourvu de composés organiques absorbeurs d'ultraviolets en sus de ceux présents dans les constituants de base du film.

5. Dispositif d'administration transcutanée de médicament selon l'une quelconque des revendications précédentes, dans lequel le composé inorganique absorbeur d'ultraviolets est choisi dans le groupe constitué de dioxyde de titane, oxyde de zinc et oxyde de fer.

6. Dispositif d'administration transcutanée de médicament selon la revendication 1, dans lequel la concentration en composé inorganique absorbeur d'ultraviolets est inférieure à 1 pour cent en poids du poids total du film translucide.

7. Dispositif d'administration transcutanée de médicament selon l'une quelconque des revendications précédentes, dans lequel le film translucide comprend du polyéthylène, des copolymères de polyéthylène ou des mélanges de polyéthylène.

8. Dispositif d'administration transcutanée de médicament selon l'une quelconque des revendications précédentes, dans lequel l'absorbance du film translucide à une longueur d'onde de 500 nanomètres est inférieure à 2 unités d'absorbance.

9. Dispositif d'administration transcutanée de médicament selon l'une quelconque des revendications précédentes, dans lequel l'absorbance du film translucide à une longueur d'onde de 300 nanomètres est supérieure à 3 unités d'absorbance.
